# EUROPÄISCHE PATENTANMELDUNG

(11) **EP 0 799 824 A1**
(43) Veröffentlichungstag der Anmeldung: **08.10.1997**
(21) Anmeldenummer: 97105080.2
(22) Anmeldetag: 26.03.1997
(51) Int. Cl.: C07D 207/46

(54) **Verfahren zur Herstellung von Carbonsäuresuccinimidylestern**

(30) Priorität: 04.04.1996 AT 609/96
(71) Anmelder: DSM Chemie Linz GmbH, A-4021 Linz (AT)
(72) Erfinder: Pöchlauer, Peter, Dr., 4040 Puchenau (AT); Praus, Antonia, 4020 Linz (AT); Hendel, Wolfram, Dr., 4060 Leonding (AT); Summer, Gerald, 4020 Linz (AT); Burger, Christian, 4060 Leonding (AT); Lamplmayr, Anita, 4020 Linz (AT); Pöschko, Harald, 4482 Ennsdorf (AT)
(74) Vertreter: Kunz, Ekkehard, Dr.

(57) **Zusammenfassung**

Verfahren zur Herstellung von Carbonsäuresuccinimidylestern durch Umsetzung von N-Hydroxysuccinimid mit einer Carbonsäure und einem Halogenphosphorsäureester der Formel 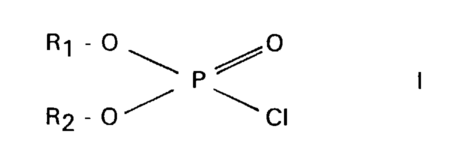 in der R₁ und R₂ gleich oder verschieden sind und einen C₂ bis C₆ Alkylrest oder einen Phenylrest bedeuten, oder R₁ und R₂ gemeinsam einen C₆-Arylrest bilden,
in Anwesenheit einer Base in einem Verdünnungsmittel bei einer Temperatur von 0°C bis zu 100°C und Isolation des entsprechenden Carbonsäuresuccinimidylesters.

## Beschreibung

Succinimidylester finden u. a. als aktivierte Acylderivate Verwendung, insbesondere wenn andere Acylierungsmittel, wie Säurechloride oder Anhydride, aufgrund der Labilität von Ausgangs- oder Endprodukten nicht eingesetzt werden können. Dies gilt besonders auf dem Gebiet der Peptidchemie. Zur Knüpfung einer kovalenten Peptidbindung ist eine aktivierte Carboxy-Komponente notwendig. Die bisher am häufigsten angewandte Methode zur Carboxy-Aktivierung beruht auf der Bildung von Succinimidylestern durch Umsetzung der entsprechenden Carbonsäure mit Carbodiimiden, wie etwa N,N'-Dicyclohexylcarbodiimid (DCC), und N-Hydroxysuccinimid. Der Nachteil bei diesem Verfahren liegt einerseits im Kopplungsreagens DCC aufgrund seiner Allergenität und des hohen Preises, und andererseits im dabei anfallenden Nebenprodukt N,N'-Dicyclohexylharnstoff, das erstens den Succinimidylester verunreinigt und zweitens entsorgt werden muß.
Aus diesen Gründen wurde versucht, das System DCC / N-Hydroxysuccinimid zu ersetzen. Mögliche Alternativen stellen Systeme vom Typ Halogenphosphorsäureester / N-Hydroxysuccinimid / Base dar.

Aus Tetrahedron Letters Vol. 21, S. 1467 - 1468, [1980] bzw. aus Chemical Abstracts Vol. 95: 203746 ist ein Verfahren zur Herstellung von Diphenylsuccinimidoylphosphat (SDPP) bekannt, bei welchem Diphenylchlorphosphat mit Hydroxysuccinimid unter den Reaktionsbedingungen für Schotten-Baumann-Reaktionen in einer wäßrigen oder organischen Lösung, beispielsweise in Methylenchlorid bei Raumtemperatur umgesetzt wird. SDPP wird isoliert, gegebenenfalls umkristallisiert und anschließend mit einer Z- oder Boc-geschützten Aminosäure in Anwesenheit einer Base und von Acetonitril zu dem entsprechenden Aminosäure-Succinimidylester, der als aktivierte Carboxy-Komponente für Peptidkopplungen Verwendung findet, umgesetzt.
Aus J. Org. Chem., Vol. 47, No 15, [1982], Seite 2985 ist ein gegenüber dem oben angeführten Verfahren verbessertes Verfahren zur Herstellung von SDPP bekannt, bei welchem SDPP unter noch vorsichtigeren Bedingungen, nämlich bei Kühlung mittels einer Eis / Kochsalzlösung, hergestellt wird.

Aufgabe der vorliegenden Erfindung war es, ein Verfahren zur Herstellung von Succinimidylestern zu finden, das auf einfache Weise und ohne Isolation von SDPP zu höheren Gesamtausbeuten an den gewünschten Succinimidylestern führt und auf eine Vielzahl von Carbonsäuren anwendbar ist.

Unerwarteterweise konnte diese Aufgabe durch ein Verfahren gelöst werden, bei welchem die Umsetzung zu den Carbonsäuresuccinimidylestern als Eintopfreaktion und bei Temperaturen bis zu 100°C durchgeführt wird, wobei die Zugabe der Reaktanten in beliebiger Reihenfolge erfolgen kann.

Gegenstand der vorliegenden Erfindung ist demnach ein Verfahren zur Herstellung von Carbonsäuresuccinimidylestern, das dadurch gekennzeichnet ist, daß N-Hydroxysuccinimid mit einer Carbonsäure und einem Halogenphosphorsäureester der Formel 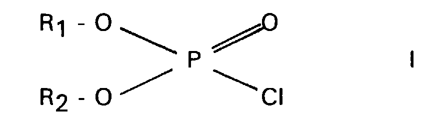 in der R₁ und R₂ gleich oder verschieden sind und einen C₂ bis C₆ - Alkylrest oder einen Phenylrest bedeuten, oder R₁ und R₂ gemeinsam einen C₆-Arylrest bilden,
in Anwesenheit einer Base in einem Verdünnungsmittel bei einer Temperatur von 0°C bis zu 100°C umgesetzt wird und der entsprechende Carbonsäuresuccinimidylester isoliert wird.

Das erfindungsgemäße Verfahren eignet sich zur Herstellung von Succinimidylestern von Carbonsäuren. Unter Carbonsäuren sind dabei alle Verbindungen der allgemeinen Formel

A - COOH II

zu verstehen, die einen unter den erfindungsgemäßen Reaktionsbedingungen stabilen Succinimidylester bilden.

Von besonderer Bedeutung sind dabei Succinimidylester von N-substituierten Aminosäuren.

In der Formel II kann der Rest A eine gesättigte oder ein- oder mehrfach ungesättigte, lineare, verzweigte oder cyclische Alkylgruppe, eine Arylgruppe, eine Arylalkylgruppe oder eine heterocyclische Gruppe bedeuten.

Der Rest A kann sowohl unsubstituiert als auch ein- oder mehrfach durch unter den Reaktionsbedingungen inerte Gruppen substituiert sein. Als Substituenten kommen beispielsweise Halogen, wie Chlor oder Brom, Hydroxygruppen, Nitrogruppen, (C₁ - C₄) Alkylgruppen, (C₁ bis C₄) Alkoxygruppen, Oxogruppen, Estergruppen oder sekundäre oder tertiäre Amingruppen in Frage. Weiters können die Verbindungen noch eine zweite Carboxygruppe enthalten, sodaß es zur Bildung von Disuccinimidylestern kommt.

Beispiele für geeignete Carbonsäuren sind:
- lineare, verzweigte oder cyclische, aliphatische, gesättigte, unsubstituierte Mono- oder Dicarbonsäuren mit 2 bis 60 C-Atomen, wie etwa Essigsäure, Propionsäure, Buttersäure, Isobuttersäure, Valeriansäure, Isovaleriansäure, Trimethylessigsäure, Ethyl-methylessigsäure, Capronsäure, Caprylsäure, Caprinsäure, Laurinsäure, Palmitinsäure, Stearinsäure, Pivalinsäure, n-Hexacosansäure,
   Malonsäure, Bernsteinsäure, Glutarsäure, Adipinsäure, Pimelinsäure, Suberinsäure, Azelainsäure, Sebacinsäure,
- lineare oder verzweigte, aliphatische, ein- oder mehrfach ungesättigte, unsubstituierte Mono- oder Dicarbonsäuren mit 2 bis 20 C-Atomen, wie etwa Acrylsäure, Methacrylsäure, Crotonsäure, Isocrotonsäure, Vinylessigsäure, Ölsäure, Sorbinsäure, Linolsäure, Linolensäure,
   Maleinsäure, Fumarsäure, Acetylendicarbonsäure, Hexindisäure, Hexendisäure,
- unsubstituierte, aromatische oder araliphatische Mono- oder Dicarbonsäuren mit 7 bis 20 C-Atomen, wie etwa Benzoesäure, Phenylessigsäure, Zimtsäure, Phthalsäure, Isophthalsäure, Terephtalsäure, 1- oder 2-Naphthalincarbonsäure, 1,5-Naphthalindicarbonsäure
- oder unsubstituierte heterocyclische Mono- oder Dicarbonsäuren wie etwa 4-Pyridincarbonsäure, 2,3-Pyridindicarbonsäure, Chinaldinsäure.

Substituierte Carbonsäuren sind beispielsweise
- Halogencarbonsäuren, wie etwa Monochloressigsäure, Chlorpropionsäure, Dichloressigsäure, Trichloressigsäure, Chlorvaleriansäure,
- Hydroxycarbonsäuren wie etwa Milchsäure, γ-Hydroxyvaleriansäure, Glykolsäure, Traubensäure, Salicylsäure, Gallussäure, p-Cumarsäure, Kaffeesäure, Mandelsäure,
- Oxocarbonsäuren wie etwa Glyoxylsäure, Brenztraubensäure, Acetessigsäure, Lävulinsäure, Pulvinsäure, E-9-Oxo-2-decensäure,
- N-substituierte Aminosäuren, wie etwa Z-Phenylalanin, Z-Asparaginsäure-β-benzylester, N-Phenylglycin, Z-Valin, N-(1-Ethoxycarbonyl-3-phenylpropyl)alanin,
sowie weitere durch NO₂, sek. oder tert. Amingruppen, Estergruppen, (C₁ bis C₄)Alkyl- oder Alkoxygruppen, substituierte Carbonsäuren, beispielsweise Nitrobenzoesäure, Acetylsalicylsäure, Toluylsäure, u.s.w..

Bei dem erfindungsgemäßen Verfahren wird die entsprechende Carbonsäure mit N-Hydroxysuccinimid und einem Halogenphosphorsäureester der Formel I in Gegenwart einer Base umgesetzt.
Als Halogenphosphorsäureester eignen sich Verbindungen der Formel I, in der R₁ und R₂ gleich oder verschieden sind und einen C₂ bis C₆ - Alkylrest oder einen Phenylrest bedeuten, oder R₁ und R₂ gemeinsam einen C₆-Arylrest bilden.
Beispiele dafür sind Diphenylchlorphosphat, Diethylchlorphosphat, Dibutylchlorphosphat und Resorcinylchlorphosphat. Bevorzugt wird Diphenyl- oder Dibutylchlorphosphat, besonders bevorzugt Diphenylchlorphosphat eingesetzt.

Als Base für die Umsetzung eignen sich Carbonate wie etwa Natriumcarbonat, Natriumhydrogencarbonat, Kaliumcarbonat u.s.w., Hydroxyde, wie etwa Natriumhydroxyd, Kaliumhydroxyd, u.s.w., oder Amine wie etwa Triethylamin, N-Methylpyrrolidon, N - Methyl- oder N - Ethylmorpholin u.s.w..

Bei der Umsetzung kann die Reihenfolge der Zugabe der einzelnen Reaktionskomponenten beliebig variieren. So können beispielsweise zuerst nur eine oder zwei der Reaktionskomponenten in einem Verdünnungsmittel vorgelegt werden. Die Base kann dabei entweder gleichzeitig mit diesen Verbindungen vorgelegt, aber auch anschließend in die Vorlage zudosiert werden. Zuletzt erfolgt die Zugabe der noch fehlenden Reaktionspartner, bevorzugt in dem Verdünnungsmittel das auch als Vorlage dient, in beliebiger Reihenfolge. Beispielsweise kann jedoch auch zuerst die Base in einem geeigneten Verdünnungsmittel vorgelegt, danach N-Hydroxysuccinimid und der Halogenphosphorsäureester unter Rühren zugegeben, und im Anschluß daran die entsprechende Carbonsäure in einem Verdünnungsmittel, das auch als Vorlage dient, zugemischt werden. Es kann aber auch zuerst die Base, N-Hydroxysuccinimid und die Carbonsäure in die Vorlage eingebracht und im Anschluß daran der Halogenphosphorsäureester, wiederum bevorzugt in dem Verdünnungsmittel das auch als Vorlage dient, dem Reaktionsgemisch zugegeben werden.

Die Umsetzung erfolgt bevorzugt mit äquivalenten Mengen der Reaktanten, ein Überschuß an N-Hydroxysuccinimid, Halogenphosphorsäureester und/oder Base kann hilfreich sein.
Pro Mol Carboxylgruppe werden somit bevorzugt 1 bis 2 Mol, besonders bevorzugt 1 bis 1,5 Mol an N - Hydroxysuccinimid, bevorzugt 1 bis 2 Mol, besonders bevorzugt 1 bis 1,5 Mol an Halogenphosphorsäureester und bevorzugt 2 bis 5 Mol, besonders bevorzugt 2 bis 4 Mol an Base zugesetzt.
Größere Mengen an N - Hydroxysuccinimid, Base oder Halogenphosphorsäureester können gewünschtenfalls auch eingesetzt werden.

Als Verdünnungsmittel können sowohl mit Wasser kaum oder nicht mischbare organische Verdünnungsmittel wie etwa Ethylacetat, Methylenchlorid, Methyl-tert. butylether u.s.w., aber auch mit Wasser mischbare organische Verdünnungsmittel wie etwa Aceton, Acetonitril, Tetrahydrofuran, Dimethoxyethan u.s.w. verwendet werden. Wird ein mit Wasser mischbares Verdünnungsmittel eingesetzt, so kann gegebenenfalls auch ein Wasser/Verdünnungsmittelgemisch verwendet werden. Die Wahl des Lösungsmittel ist dabei abhängig von den Eigenschaften der eingesetzten Reaktanten und des gewünschten Endproduktes.

Die Reaktionstemperatur kann 0°C bis 100°C betragen. Bevorzugt liegt die Reaktionstemperatur zwischen 10 und 80°C, besonders bevorzugt zwischen 20 und 60°C. Tiefere Temperaturen können gewünschtenfalls auch eingestellt werden, die Reaktion läuft dann jedoch langsamer ab.
Bei der Umsetzung kann es zu einer Erhöhung der Temperatur des Reaktionsgemisches kommen, es kann jedoch auch durch externe Energiezufuhr die gewünschte Reaktionstemperatur eingestellt werden.

In Abhängigkeit von der chemischen Struktur der jeweils eingesetzten Carbonsäuren wird das so erhaltene Reaktionsgemisch zur Vervollständigung der Reaktion für einige Minuten bis zu mehreren Stunden auf der Reaktionstemperatur gehalten. Anschließend wird das Reaktionsgemisch, falls erforderlich, abgekühlt, bevorzugt auf etwa 15 bis 30°C, und der Carbonsäuresuccinimidylester gewünschtenfalls isoliert.

Die Isolierung des nach obigem Verfahren erhaltenen Carbonsäuresuccinimidylesters kann in Abhängigkeit von den Eigenschaften des Succinimidylesters variieren.
Wurde die Umsetzung in einem mit Wasser nicht oder kaum mischbaren Verdünnungsmittel durchgeführt, so kann das nach dem erfindungsgemäßen Verfahren erhaltene Reaktionsgemisch zur Abtrennung von Salzen mit Wasser versetzt werden, worauf es zur Phasentrennung kommt und der entsprechende Carbonsäuresuccinimidylester aus der organischen Phase isoliert wird. Zur Isolierung des Esters aus der organischen Phase wird diese auf übliche Weise von der wäßrigen Phase abgetrennt, gewaschen und gegebenenfalls getrocknet, anschließend wird das Verdünnungsmittel entfernt, etwa durch Destillation, gegebenenfalls unter vermindertem Druck.

Wurde für die Umsetzung ein mit Wasser mischbares Verdünnungsmittel verwendet, so ist das Verdünnungsmittel zuerst zu entfernen. Anschließend wird der verbleibende Rückstand mit einem Gemisch aus Wasser und einem mit Wasser nicht oder kaum mischbaren Verdünnungsmittel versetzt, worauf es zur Phasentrennung kommt und der Ester aus der organischen Phase, wie oben beschrieben, isoliert wird.
Alternativ kann durch Zufügen eines geeigneten Verdünnungsmittels, etwa einer wäßrigen Lösung eines anorganischen Salzes, beispielsweise Natriumchlorid oder Natriumsulfat, eine Phasentrennung und damit eine Trennung des Succinimidylesters von Nebenprodukten herbeigeführt werden.

Das so erhaltene Rohprodukt kann gewünschtenfalls noch durch übliche Verfahren wie etwa Umkristallisieren, Chromatographie gereinigt werden.
Bei einer weiteren Isolierungsvariante wird beispielsweise das nach der Umsetzung erhaltene Reaktionsgemisch eingedampft und der Rückstand in einem geeigneten Verdünnungsmittel, wie etwa Ethanol, aufgenommen, wobei die anderen Reaktionsprodukte in Lösung gehen und der Carbonsäuresuccinimidylester als Feststoff zurückbleibt. Der Succinimidylester wird sodann abfiltriert, nachgewaschen oder umkristallisiert und getrocknet.

Das nach dem erfindungsgemäßen Verfahren erhaltene Reaktionsgemisch kann jedoch auch ohne Aufarbeitung bzw. Isolierung des Carbonsäuresuccinimidylesters für eine Folgereaktion, beispielsweise für eine Peptidkopplungsreaktion oder für andere Acylierungsreaktionen mit geeigneten Nucleophilen, weiterverwendet werden.
So kann beispielsweise die den Carbonsäuresuccinimidylester enthaltende organische Phase ohne vorherige Aufarbeitung direkt nach Abtrennung von der die anderen Reaktionsprodukte enthaltenden wäßrigen Phase für den nächsten Schritt eingesetzt werden. In Abhängigkeit von der jeweiligen Folgereaktion wird dazu gegebenenfalls das Verdünnungsmittel der organischen Phase entfernt, der ungereinigte Carbonsäuresuccinimidylester in einem für die jeweilige Folgereaktion geeigneten Verdünnungsmittel gelöst und mit einem entsprechenden Nucleophilen, beispielsweise mit einer Aminosäure, einem Aminosäurederivat oder einem Amin, umgesetzt, worauf das gewünschte Folgeprodukt aus dem Reaktionsgemisch isoliert wird. Die einzustellenden Reaktionsparameter sind von der jeweils gewählten Folgereaktion abhängig und können daher in weiten Bereichen variieren.
Bevorzugt wird als Folgereaktion eine Peptidkopplung durchgeführt. Dabei wird der Carbonsäuresuccinimidylester mit der Aminogruppe einer Aminosäure oder einem Aminosäurederivat unter Abspaltung von N-Hydroxysuccinimid zu dem entsprechenden Peptid gekoppelt.

Durch das erfindungsgemäße Verfahren werden Carbonsäuresuccinimidylester und deren Folgeprodukte in einer einfachen Eintopfreaktion in hohen Ausbeuten und in ausgezeichneter Reinheit erhalten.

### Beispiel 1:

In einem 3-Halsrundkolben mit Tropftrichter, Thermometer, KPG-Rührer und mit einem Kühler mit Ableitungsrohr wurden 3,45 g (0,041 mol) Natriumhydrogencarbonat, 0,10 g H₂O und 30 ml Aceton vorgelegt. Danach wurden 2,79 g (0,010 mol) (S,S)-N-(1-Ethoxycarbonyl-3-phenylpropyl)alanin (ECPA) und 1,20 g (0,010 mol) N-Hydroxysuccinimid zugegeben und das Reaktionsgemisch auf 50°C erhitzt. Innerhalb von 10 Minuten wurden anschließend 3,22 g (0,012 mol) Diphenylchlorphosphat zugetropft und das so erhaltene Reaktionsgemisch 1 Stunde bei 50°C gerührt. Im Anschluß daran wurde im Wasserbad auf 20°C abgekühlt und das Aceton im Wasserstrahlvakuum über den Rückflußkühler im Reaktionskolben abgezogen. Zum Rückstand wurden sodann 30 ml Ethylacetat und 30 ml H₂0 zugegeben. Nach erfolgter Phasentrennung im Schütteltrichter wurde die organische Phase mit 30 ml NaHCO₃-Lösung extrahiert, mit Na₂SO₄ p.a. getrocknet und bei 40°C und 20 mbar einrotiert.

Ausbeute an ECPA-Succinimidylester: 3,25 g ( 86,3 % d. Th.)

### Beispiel 2:

Es wurden 1,4 g (0,012 mol) N-Hydroxysuccinimid, 3,5 g (0,041 mol) Natriumhydrogencarbonat und 10 ml Ethylacetat vorgelegt. Anschließend wurden 3,8 g (0,014 mol) Diphenylchlorphosphat in 10 ml Ethylacetat zugetropft. Danach wurde eine Suspension von 2,8 g (0,010 mol) ECPA in 30 ml Ethylacetat bei 50°C zugegeben und 1,5 Stunden bei Raumtemperatur gerührt. Zu dem so erhaltenen Raktionsgemisch wurden 30 ml dest. H₂O zugegeben, worauf eine Phasentrennung erfolgte. Die organische Phase wurde sodann einmal mit 30 ml Natriumhydrogencarbonat extrahiert, getrocknet und bei ca. 50°C und 20 mbar einrotiert.

Ausbeute an ECPA-Succinimidylester: 3,0 g (80% d. Th.)

### Beispiel 3:

2,42 g (0,021 mol) N-Hydroxysuccinimid und 5,06 g (0,050 mol) Triethylamin wurden in 5 ml Ethylacetat vorgelegt und bei Raumtemperatur gerührt. Zu dieser Suspension wurden 2,72 g (0,020 mol) feste 4-Toluylsäure in 5 Min. zugegeben und anschließend eine Lösung von 5,64 g (0,021 mol) Diphenylchlorphosphat innerhalb von 20 Min. zugetropft, wobei sich das Reaktionsgemisch auf ca. 35°C erwärmte.
Bis zur Vollständigkeit der Reaktion wurde 1 Stunde bei Raumtemperatur gerührt. Dann wurde das Reaktionsgemisch mit 100 ml Ethylacetat verdünnt und die organische Phase mit je 50 ml Wasser, 2 N-Salzsäure und 10 %iger wäßriger Natriumhydrogencarbonatlösung gewaschen und anschließend eingedampft. Es wurden 4,65 g Rohprodukt isoliert, das anschließend mit 20 ml Methyl-tert. butylether 30 Minuten bei Raumtemperatur gerührt wurde. Der weiße kristalline Festkörper wurde abfiltriert und bei 50°C und 10 mbar getrocknet.
Es wurden 4,28 g (94 %) 4-Toluylsäuresuccinimidylester isoliert.
Fp.: 176 - 180°C (Zers.)

### Beispiel 4:

1,73 g (0,010 mol) Chinaldinsäure wurden in 30 ml Aceton vorgelegt und 1,27 g (0,011 mol) festes N-Hydroxysuccinimid zugegeben. Anschließend wurde zu dieser Suspension eine Lösung von 2,53 g (0,025 mol) Triethylamin in 10 ml Aceton in 5 Min. zugegeben und bei Raumtemperatur gerührt. Nach 30 Min. wurde eine Lösung von 3,22 g (0,012 mol) Diphenylchlorphosphat in 10 ml Aceton innerhalb von 10 Min. zugetropft, wobei sich die Lösung unter leicher Erwärmung rot verfärbte und Salze ausfielen. Bis zur Vollständigkeit der Reaktion wurde 20 Stunden bei Raumtemperatur gerührt. Die so erhaltene Suspension wurde eingedampft, in 50 ml Ethanol aufgenommen und bei Raumtemperatur 1 Stunde gerührt. Dann wurde der Festkörper abfiltriert, mit 2 x 3 ml Ethanol nachgewaschen und 1 Stunde bei 1 mbar getrocknet. Es wurden 2,45 g (91 %) eines rosa gefärbten Pulvers an Chinaldinsäuresuccinimidylester erhalten.

Fp.: 193 - 196°C (Zers.)

### Beispiel 5:

1,38 g (0,012 mol) N-Hydroxysuccinimid und 3,36 g (0,040 mol) Natriumhydrogencarbonat wurden in 10 ml Acetonitril vorgelegt und bei Raumtemperatur gerührt. Es wurde eine Lösung von 1,73 g (0,010 mol) Chinaldinsäure in 80 ml Acetonitril und eine Lösung von 3,22 g (0,012 mol) Diphenylchlorphosphat in 10 ml Acetonitril innerhalb von 30 Min. zugegeben. Die erhaltene, rosa gefärbte Suspension wurde 16 Stunden bei Raumtemperatur gerührt und anschließend noch 24 Stunden im Wasserbad auf 40°C erwärmt.
Nach Beendigung der Reaktion wurde das Reaktionsgemisch im Vakuum eingedampft und der Rückstand in 50 ml Dichlormethan aufgenommen. Die Suspension wurde filtriert und der Filterrückstand mit 2 x 20 ml Dichlormethan nachgewaschen. Die vereinigten Filtrate wurden mit je 100 ml Wasser, 5 %iger wäßriger NaHCO₃-Lösung und nochmals Wasser gewaschen. Nach Eindampfen der organischen Phase wurden 2,0 g Rohprodukt erhalten. Dieses wurde in einem Gemisch aus 30 ml Dichlormethan und 150 ml n-Hexan umgefällt. Der Niederschlag wurde filtriert, mit 5 ml n-Hexan nachgewaschen und im Vakuum getrocknet.
Es wurden 1,9 g (70 %) eines beigen Feststoffes an Chinaldinsäuresuccinimidylester erhalten.

Fp.: 191 - 196 °C (Zers.)

### Beispiel 6:

1,73 g (0,010 mol) Chinaldinsäure und 1,27 g (0,011 mol) N-Hydroxysuccinimid wurden in 30 ml Acetonitril vorgelegt und bei Raumtemperatur gerührt. Diese Suspension wurde innerhalb von 10 Min. mit einer Lösung von 2,53 g (0,025 mol) Triethylamin in 10 ml Acetonitril versetzt und 30 Min. bei Raumtemperatur gerührt. Dann wurde eine Lösung von 3,22 g (0,012 mol) Diphenylchlorphosphat in 10 ml Acetonitril innerhalb von 10 Min. zugetropft. Die erhaltene, tiefrot gefärbte Suspension wurde 20 Stunden bei Raumtemperatur gerührt. Nach Beendigung der Reaktion wurde das Reaktionsgemisch im Vakuum eingedampft und der violette Rückstand in 100 ml Dichlormethan aufgenommen. Die organische Phase wurde mit je 100 ml Wasser, 1 N-Salzsäure und verd. NaHCO₃-Lösung gewaschen. Nach Eindampfen der organischen Phase wurden 2,55 g Rohprodukt erhalten. Dieses wurde in 25 ml Ethanol 2,5 Stunden ausgerührt. Der Rückstand wurde abfiltriert, mit 4 ml Ethanol nachgewaschen und im Vakuum getrocknet. Es wurden 1,85 g (69 %) eines violetten Feststoffes an Chinaldinsäuresuccinimidylester erhalten.

Fp.: 190 - 194 °C (Zers.)

### Beispiel 7:

1,73 g (0,010 mol) Chinaldinsäure und 1,27 g (0,011 mol) N-Hydroxysuccinimid wurden in 30 ml Aceton vorgelegt und 1,27g (0.011 mol) festes N - Hydroxysuccinimid zugegeben. Diese Suspension wurde innerhalb von 5 Min. mit einer Lösung von 2,48 g (0,025 mol) 1-Methyl-2-pyrrolidon in 10 ml Aceton versetzt und 30 Min. bei Raumtemperatur gerührt. Dann wurde eine Lösung von 3,22 g (0,012 mol) Diphenylchlorphosphat in 10 ml Aceton zugetropft. Die erhaltene Suspension wurde 69 Stunden bei Raumtemperatur gerührt. Nach Beendigung der Reaktion wurde das gelbliche Reaktionsgemisch eingedampft und der Rückstand in 100 ml Dichlormethan aufgenommen und die organische Phase mit je 60 ml Wasser, 2N-Salzsäure, ges. NaHCO₃-Lösung und nochmals Wasser gewaschen. Nach Eindampfen der organischen Phase wurden der verbleibende Rückstand in 20 ml Ethanol 30 Minuten ausgerührt, abfiltriert, mit 3 ml Ethanol nachgewaschen und 4 Stunden bei 10 mbar und 30°C getrocknet. Es wurden 0,95 g (35 %) eines violetten Feststoffes an Chinaldinsäuresuccinimidylester erhalten.

Fp.: 192 - 196 °C (Zers.)

### Beispiel 8:

2,51 g (0,010 mol) Z-Valin wurden in 30 ml Ethylacetat vorgelegt und 1,27 g (0,011 mol) festes N-Hydroxysuccinimid zugegeben. Diese Suspension wurde mit einer Lösung von 2,53 g (0,025 mol) Triethylamin in 10 ml Ethylacetat versetzt und 30 Min. bei Raumtemperatur gerührt. Dann wurde eine Lösung von 3,22 g (0,012 mol) Diphenylchlorphosphat in 10 ml Ethylacetat zugetropft. Aus der zuvor klaren Lösung bildete sich dabei unter leichter Erwärmung auf 32°C eine dicke Suspension, die 23 Stunden bei Raumtemperatur gerührt und dann filtriert wurde. Der Filterrückstand wurde zweimal mit je 10 ml Ethylacetat nachgewaschen. Die vereinigten Filtrate wurden mit je 25 ml Wasser und 5 %iger NaHCO₃-Lösung, sowie nochmals mit zweimal 25 ml Wasser gewaschen. Nach der Phasentrennung wurde die Ethylacetat-Phase über Natriumsulfat getrocknet, filtriert und das Verdünnungsmittel im Vakuum entfernt. Es wurden 3,25 g eines weißen Feststoffes erhalten. 2,98 g davon wurden aus einem Gemisch von 5 ml Dichlormethan und 15 ml Diisopropylether umgefällt, worauf 2,41 g (76%) feine weiße Kristalle an Z-Valinsuccinimidylester erhalten wurden.

Fp.: 117 - 120°C

### Beispiel 9:

5,99 g (0,020 mol) Z-Phenylalanin, 2,42 g (0,021 mol) N-Hydroxysuccinimid und 6,72 g (0,080 mol) festes Natriumhydrogencarbonat wurden in 20 ml Ethylacetat vorgelegt und bei Raumtemperatur gerührt. Dann wurde eine Lösung von 5,64 g (0,021 mol) Diphenylchlorphosphat in 40 ml Ethylacetat innerhalb von 20 Minuten zugetropft, wobei sich die Suspension eindickte. Nach Verdünnung mit 50 ml Ethylacetat wurde das Reaktionsgemisch 2 Stunden auf 50 °C erwärmt, bis mittels DC-Kontrolle keine weitere Umsetzung festgestellt wurde. Das Reaktionsgemisch wurde mit je 50 ml Wasser, ges. NaHCO₃-Lösung und nochmals mit Wasser gewaschen. Nach erfolgter Phasentrennung wurde die Ethylacetat-Phase im Vakuum bei 50°C eingedampft. Es wurden 4,81 g kristallines Rohprodukt erhalten, das 2 Stunden bei Raumtemperatur mit 20 ml Methyl-tert. butylether ausgerührt wurde. Die Ausbeute an Z-Phenylalaninsuccinimidylester betrug 4,29 g (54%)

Fp.: 135 - 139°C

### Beispiel 10:

1,07 g (0,0030 mol) Z-Asparaginsäure-β-benzylester wurden in 10 ml Ethylacetat vorgelegt und 0,38 g (0,0033 mol) festes N-Hydroxysuccinimid zugegeben. Anschließend wurde zu dieser Suspension eine Lösung von 0,76 g (0,0075 mol) Triethylamin in Ethylacetat zugegeben und 10 Minuten bei Raumtemperatur gerührt. Danach wurde eine Lösung von 0,97 g (0,0075 mol) Diphenylchlorphosphat in 3 ml Ethylacetat zugetropft, wodurch aus der zuvor klaren Lösung unter leichter Erwärmung auf max. 32°C eine dicke Suspension entstand. Diese wurde zur Vervollständigung der Reaktion 24 Stunden bei Raumtemperatur gerührt und dann filtriert. Der Filterrückstand wurde zweimal mit je 3 ml Ethylacetat nachgewaschen. Die vereinigten Filtrate wurden je zweimal mit 25 ml Wasser und 5 %iger NaHCO₃-Lösung und dann nochmals mit 50 ml Wasser gewaschen. Nach der Phasentrennung wurde die Ethylacetat-Phase über Natriumsulfat getrocknet, filtriert und das Verdünnungsmittel im Vakuum entfernt. Es wurden 1,37 g eines farblosen, harzigen Rohproduktes isoliert, die mit einem Gemisch aus 2,5 ml Dichlormethan und 25 ml Diisopropylether, sowie 10 ml Hexan ausgerührt und anschließend durch Erwärmen in 50 ml Diisopropylether auf 40°C zur Kristallisation gebracht wurden. Nach dem Abfiltrieren, Waschen mit 2 x 5 ml Diisopropylether und Trocknen im Vakuum wurden 0,98 g (72%) weißer, kristalliner Z-Asparaginsäure-β-benzylestersuccinimidylester erhalten.

Fp.: 82 - 84°C

### Beispiel 11:

0,72 g (0,010 mol) Acrylsäure wurden in 30 ml Aceton vorgelegt und 1,27 g (0,011 mol) festes N-Hydroxysuccinimid zugegeben. Die entstandene Lösung wurde mit 3,36 g (0,040 mol) festem Natriumhydrogencarbonat versetzt und die so erhaltene weiße Suspension bei Raumtemperatur gerührt. Dann wurde eine Lösung von 3,22 g (0,012 mol) Diphenylchlorphosphat in 10 ml Aceton zugetropft und das Reaktionsgemisch zur Vervollständigung der Reaktion 24 Stunden bei Raumtemperatur und weitere 3 Stunden bei 50°C gerührt, wobei die eindickende Suspension mit 25 ml Aceton verdünnt wurde. Anschließend wurde das Verdünnungsmittel im Vakuum entfernt und der Rückstand in 60 ml Dichlormethan aufgenommen. Nicht gelöster Feststoff wurde filtriert und zweimal mit je 10 ml Dichlormethan gewaschen. Die vereinigten Dichlormethan-Phasen wurden mit 40 ml Wasser gewaschen und nach der Phasentrennung im Vakuum eingedampft. Es wurden 1,53 g festes Rohprodukt erhalten, welches 3 Stunden mit 15 ml Diisopropylether ausgerührt wurde. Der verbleibende weiße Feststoff wurde abfiltriert, zweimal mit je 3 ml Diisopropylether gewaschen und im Vakuum getrocknet. Es wurden 1,1 g (65%) an Acrylsäuresuccinimidylester erhalten.

Fp.: 63 - 68°C

### Beispiel 12:

1,27 g (0,011 mol) N-Hydroxysuccinimid wurden in 10 ml Ethylacetat vorgelegt, eine Lösung von 3,22 g (0,012 mol) Diphenylchlorphosphat in 10 ml Ethylacetat zugegeben und anschließend eine Lösung von 2,53 g (0,025 mol) Triethylamin in 10 ml Ethylacetat innerhalb von 10 Minuten zugetropft, wobei die Temperatur auf 41°C anstieg. Es entstand eine dicke, weiße Suspension, welche mit einer Lösung von 0,6 g (0,01 mol) Essigsäure in 10 ml Ethylacetat versetzt und 16 Stunden auf 45-55°C erhitzt wurde. Nach Beendigung der Reaktion wurde der Feststoff abfiltriert und mit 2 x 3 ml Ethylacetat nachgewaschen. Die vereinigten Filtrate wurden im Vakuum eingedampft und der verbleibende semikristalline Rückstand mit 20 ml Ethanol 30 Minuten ausgerührt. Der so erhaltene weiße Feststoff wurde abfiltriert, mit 2 x 3 ml Ethanol gewaschen, trockengesaugt und anschließend mit 10 ml Isopropanol ausgerührt. Nach dem Abfiltrieren, Waschen mit 2 x 2 ml Isopropanol und Trocknen im Vakuum wurden 1,1 g (70%) weißer Essigsäuresuccinimidylester erhalten.

Fp.:130- 134°C

### Beispiel 13:

2,42 g (0,021 mol) N-Hydroxysuccinimid, 2,72 g (0,021 mol) 4-Tolylsäure und 4,05 g (0,040 mol) Triethylamin wurden in 20 ml Ethylacetat vorgelegt und bei Raumtemperatur gerührt. Zu dieser Suspension wurde innerhalb von 20 Minuten eine Lösung von 5,64 g (0,021 mol) Diphenylchlorphosphat in 40 ml Ethylacetat zugetropft, wobei sich das Reaktionsgemisch auf ca. 35°C erwärmte. Zur Vervollständigung der Reaktion wurde noch 1 Stunde bei Raumtemperatuer gerürt. Dann wurde das Reaktionsgemisch mit 50 ml Ethylacetat verdünnt und mit je 50 ml Wasser, 2N Salzsäure, gesättigter Natriumhydrogencarbonatlösung und nochmals Wasser gewaschen. Die organische Phase wurde abgetrennt und mit 4,05 g (0,040 mol) Triethylamin und 2,62 g (0,020 mol) L-Valinmethylester versetzt. Das Reaktionsgemisch wurde 1,5 Stunden bei Raumtemperatur gerührt und anschließend 3,5 Stunden unter Rückfluß erhitzt. Nach Rühren bei Raumtemperatur über Nacht wurde eine ölige, schwerere zweite Phase abgetrennt und die Ethylacetat-Phase mit je 50 ml Wasser, ges. NaHCO₃-Lösung und noch dreimal mit je 50 ml Wasser gewaschen. Nach Entfernen des Verdünnungsmittels bei 50°C im Vakuum verblieben 3,41 g Rohprodukt. 3,01 g davon wurden 1 Stunde mit 10 ml Methyltertiärbutylether ausgerührt. Nach Filtration und Trocknung des Rückstandes im Vakuum bei 50°C wurden 2,42 g (55%) weißes, kristallines 4-Toluylsäure-L-valinmethylesteramid erhalten.

Fp.: 98 - 100°C

### Beispiel 14:

5,65 g (0,015 mol) eines nach Beispiel 1 oder 2 erhaltenen ECPA-Succinimidylesters wurden in 45 ml Ethanol gelöst und innerhalb von 5 Minuten mit einer Lösung von 3,4 g (0,029 mol) L-Prolin in 5,65 ml Wasser und dann innerhalb von 15 Minuten mit 8,3 ml (0,060 mol) Triethylamin versetzt. Die Temperatur stieg dabei von 22 auf 30°C. Anschließend wurde die Lösung über Nacht bei Raumtemperatur gerührt und dann das Ethanol bei 40°C und 20 mbar abrotiert. Der Rückstand wurde in 17 ml Wasser gelöst und danach 2 mal mit je 55 ml und einmal mit 25 ml Methyltertiärbutylether (MTBE) extrahiert. Die 3 MTBE-Phasen wurden vereinigt und bei 40°C und 20 mbar einrotiert. Die Auswaage betrug 0,90 g.
Die wäßrige Phase wurde mit 6 ml 2M Schwefelsäure von pH 6,3 auf pH 2,8 gestellt. Dann wurden 1,7 g Natriumsulfat p.a. (0,7mol bezogen auf H₂O) zugefügt. Anschließend wurde dreimal mit je 80 ml und zweimal mit je 30 ml Ethylacetat extrahiert. Die vereinigten Ethylacetat-Phasen wurden, mit Natriumsulfat getrocknet und bei 40°C und 20 mbar einrotiert.
Die Auswaage an (S,S,S)-N-((1-Ethoxycarbonyl-3-phenylpropyl)-alanyl)-prolin betrug 5,25 g (92,9%)

### Beispiel 15:

Analog Beispiel 3 wurden 2,76 g (0,024 mol) N-Hydroxysuccinimid und 5,06 g (0,050 mol) Triethylamin in 5 ml Ethylacetat vorgelegt und bei Raumtemperatur gerührt. Zu dieser Suspension wurden 2,72 g (0,020 mol) feste 4-Toluylsäure in 5 Min. zugegeben und anschließend eine Lösung von 3,47 g (0,020 mol) Diethylchlorphosphat innerhalb von 20 Min. zugetropft, wobei sich das Reaktionsgemisch auf ca. 35°C erwärmte.
Bis zur Vollständigkeit der Reaktion wurde 3 Stunden bei Raumtemperatur gerührt. Die Aufarbeitung des Reaktionsgemisches erfolgte analog Beispiel 3. Es wurden 2,8 g roher 4-Toluylsäuresuccinimidylester isoliert.

## Patentansprüche

1. Verfahren zur Herstellung von Carbonsäuresuccinimidylestern, dadurch gekennzeichnet, daß N-Hydroxysuccinimid mit einer Carbonsäure und einem Halogenphosphorsäureester der Formel 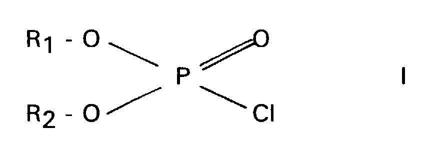 in der R₁ und R₂ gleich oder verschieden sind und einen C₂ bis C₆ Alkylrest oder einen Phenylrest bedeuten, oder R₁ und R₂ gemeinsam einen C₆-Arylrest bilden,
in Anwesenheit einer Base in einem Verdünnungsmittel bei einer Temperatur von 0°C bis zu 100°C umgesetzt wird und der entsprechende Carbonsäuresuccinimidylester isoliert wird.

2. Verfahren nach Anspruch 1, dadurch gekennzeichnet, daß als Halogenphosphorsäureester der Formel I Diphenylchlorphosphat, Diethylchlorphosphat, Dibutylchlorphosphat oder Resorcinylchlorphosphat verwendet wird.

3. Verfahren nach Anspruch 1, dadurch gekennzeichnet, daß als Base Natrium- oder Kaliumcarbonat, Natrium- oder Kaliumhydrogencarbonat, Natrium- oder Kaliumhydroxyd, Triethylamin, N-Methylpyrrolidon, N-Methyl- oder N-Ethylmorpholin verwendet wird.

4. Verfahren nach Anspruch 1, dadurch gekennzeichnet, daß als Verdünnungsmittel für die Umsetzung ein mit Wasser kaum oder nicht mischbares organisches Verdünnungsmittel oder ein mit Wasser mischbares organisches Verdünnungsmittel, gegebenenfalls in Kombination mit Wasser, eingesetzt wird.

5. Verfahren nach Anspruch 4, dadurch gekennzeichnet, daß als mit Wasser mischbares Verdünnungsmittel Aceton, Acetonitril, Tetrahydrofuran oder Dimethoxyethan verwendet wird.

6. Verfahren nach Anspruch 4, dadurch gekennzeichnet, daß als mit Wasser nicht oder kaum mischbares Verdünnungsmittel Ethylacetat, Methylenchlorid oder Methyl-tert. butylether verwendet wird.

7. Verfahren nach Anspruch 1, dadurch gekennzeichnet, daß pro Mol Carboxylgruppe mindestens 1 Mol N - Hydroxysuccinimid, mindestens 1 Mol Halogenphosphorsäureester und mindestens 2 Mol Base eingesetzt werden.

8. Verfahren nach Anspruch 1, dadurch gekennzeichnet, daß pro Mol Carboxylgruppe 1 bis 2 Mol N - Hydroxysuccinimid, 1 bis 2 Mol Halogenphosphorsäureester und 2 bis 5 Mol Base eingesetzt werden.

9. Verfahren zur Herstellung von Folgeprodukten von Carbonsäuresuccinimidylestern, dadurch gekennzeichnet, daß ein bei der Durchführung des Verfahrens nach Anspruch 1 erzeugtes Reaktionsgemisch, welches einen Carbonsäuresuccinimidylester enthält, mit einem für Acylierungen geeigneten Nucleophil weiter umgesetzt wird.

10. Verfahren nach Anspruch 9, dadurch gekennzeichnet, daß der Carbonsäuresuccinimidylester mit der Aminogruppe einer Aminosäure oder eines Aminosäurederivates unter Abspaltung von N-Hydroxysuccinimid zu einem Peptid gekoppelt wird.
